# EUROPÄISCHE PATENTSCHRIFT

(11) **EP 0 200 149 B1**
(45) Veröffentlichungstag und Bekanntmachung des Hinweises auf die Patenterteilung: **06.12.1989**
(21) Anmeldenummer: 86105586.1
(22) Anmeldetag: 23.04.1986
(51) Int. Cl.: C07D 405/06, A01N 43/653, C07D 319/06

(54) **1,3-Dioxan 5-yl-alkyltriazole, ihre Herstellung, ihre Verwendung zur Regulierung des Pflanzenwachstums und Mittel dafür**
1,3-Dioxan-5-yl alkyl triazoles, their preparation, their use as plant growth regulators and compositions therefor
1,3-Dioxane-5-yl alkyl triazoles, leur préparation, leur utilisation comme régulateur pour la croissance des plantes et composition

(30) Priorität: 27.04.1985 DE 3515309
(43) Veröffentlichungstag der Anmeldung: 10.12.1986
(73) Patentinhaber: BASF Aktiengesellschaft, 67063 Ludwigshafen (DE)
(72) Erfinder: Rentzea, Costin, Dr., D-6900 Heidelberg (DE); Sauter, Hubert, Dr., D-6800 Mannheim 1 (DE); Tuerk, Wolfgang, Dr., D-6700 Ludwigshafen (DE); Wenderoth, Bernd, Dr., D-6840 Lampertheim (DE); Richarz, Winfried, Dr., D-6081 Stockstadt (DE); Jung, Johann, Prof. Dr., D-6703 Limburgerhof (DE); Rademacher, Wilhelm, Dr., D-6703 Limburgerhof (DE)

(56) Entgegenhaltungen:
- EP-A- 0 044 407
- EP-A- 0 102 543

## Beschreibung

Die Erfindung betrifft neue 1,3-Dioxan-5-yl-alkyltriazole, Verfahren zu ihrer Herstellung sowie Mittel zur Regulierung des Pflanzenwachstums mit diesen Verbindungen.

Es ist bekannt, daß bestimmte 2-Halogenethyl-trialkylammonium-halogenide das Pflanzenwachstum regulierende Eigenschaften aufweisen (vgl. US-PS 3 156 554). So läßt sich mit Hilfe von (2-Chlorethyl)-trimethyl-ammoniumchlorid das Pflanzenwachstum beeinflussen. Allerdings ist die Wirksamkeit dieses Stoffes, vor allem bei niedrigen Aufwandmengen, nicht immer befriedigend.

Es ist ferner bekannt, 3,3-Dimethyl-2-(1,2,4-triazol-1-yl)-1-(4-Chlorbenzoyl)-butan zur Regulierung des Pflanzenwachstums zu verwenden (DE-OS 2 739 352).

Weiterhin bekannt ist die pflanzenwachstumsregulierende Wirkung von 3,3-Dimethyl-2-(1,2,4-triazol-1-yl)-1-methyl-1-(4-Chlorbenzoyl)-butan (DE-OS 2 921 168) und von 1-(5-Methyl-1,3-dioxan-5-yl)-2-(1,2,4-triazol-1-yl)-3-(4-trifluormethylphenyl)-propen-l-ol (DE-OS 3 026 140) und von N-Methyltriazolen, deren Methylgruppe erstens mit einem 1,3-Dioxan-5-yl- und zweitens einem Arylmethyl-Rest substituiert ist (EP-A 44 407).

Die Wirkung der als wachstumsregulierend bekannten Verbindungen ist, mindestens bei bestimmten Zielorganismen, hinsichtlich Aufwandmenge, Nebenwirkungsarmut und morphospezifischer Wirkung verbesserungsbedürftig.

Demgemäß wurden neue Verbindungen der Formel I gefunden, 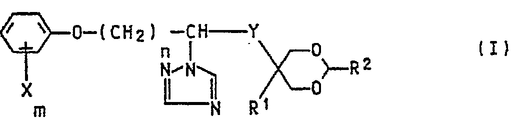 in der
R1 und R2 verschieden oder gleich sind und Wasserstoff oder Alkyl mit 1 bis 5 C-Atomen bedeuten,
X (m = null bis 5) gleiche oder verschiedene Substituenten, ausgewählt aus Halogen, Cyano, Trifluormethyl, Nitro, C₁-C₄-Alkyl, C₁-C₄-Alkoxy, Phenyl oder Wasserstoff bedeuten,
n eine ganze Zahl von 2 bis 6 und
Y die -CO- oder die -CH(OH)-Gruppe bedeutet.

Diese Verbindungen eignen sich hervorragend zur Beeinflussung des Pflanzenwachstums und weisen eine sehr gute Pflanzenverträglichkeit auf. Bei den Verbindungen handelt es sich um Diastereomere mit jeweils zwei Antipodenpaaren (Enantiomeren), die an den beiden Chiralitätszentren die Konfiguration R^{*},S^{*} oder R^{*},R^{*} haben können (vgl. D. Seebach und V. Prelog, Angew. Chem. 94, 696 (1982) und dort angegebene Literatur).

Gegenstand der vorliegenden Erfindung sind sowohl die Diastereomeren(-gemische) als auch die auf bekannte Weise erhältlichen, reinen Enantiomeren.

R1 und R2 bedeuten bevorzugt Wasserstoff, Methyl, Ethyl, n-Propyl, Isopropyl, n-Butyl, sek.-Butyl, iso-Butyl, tert.-Butyl, n-Pentyl und Neopentyl.

Xₘ- ist bevorzugt Wasserstoff, 2-Fluor-, 4-Fluor-, 2-Chlor-, 3-Chlor-, 4-Chlor-, 4-Brom-, 2,4-Dichlor-, 2,6-Dichlor-, 3,4-Dichior-, 2,4,6-Trichlor-, 2-Chlor-4-methyl-, 2-Methyl-4-chlor-, 2-Methyl-, 3-Methyl-, 4-Methyl-, 4-Ethyl-, 4-Isopropyl-, 4-tert.-Butyl-, 2,4-Dimethyl-, 2,4,6-Trimethyl-, 2-Methoxy-4-methyl-, 2-Methoxy-, 4-Methoxy-, 4-Ethoxy-, 2-Trifluormethyl-, 4-Trifluormethyl-, 4-Cyano-, 4-Nitro- und 4-Phenyl.

Die neuen Verbindungen lassen sich herstellen, indem man
a) ein 1,2,4-Triazol mit einem a-Bromketon der Formel 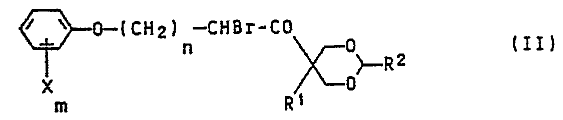 oder
b) ein Aryaloxyalkylhalogenid der Formel 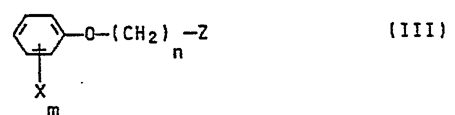 wobei Z Halogen (Chlor oder Brom) ist, mit einem 1-(1,3-Dioxan-5-yl)-2-(1,2,4-triazolyl)-ethan-1-on der Formel 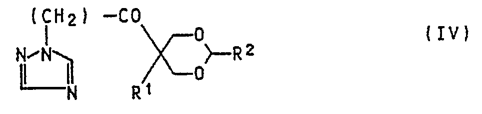 umsetzt und die so erhaltenen Verbindungen gegebenenfalls anschließend vom Keton zum sek. Alkohol reduziert.

Die Umsetzungen a) und b) können nach bekannten Verfahren durchgeführt werden, ebenso die Reduktion des Ketons zum Alkohol. Die entsprechenden Reaktionsteilnehmer sind z.T. bekannt (vgl. EP 069 290 bzw. DE-OS 3 025 879).

Die Bromketone der Formel II sind neue Verbindungen. Sie können z.B. durch Bromierung entsprechender Verbindungen der Formel 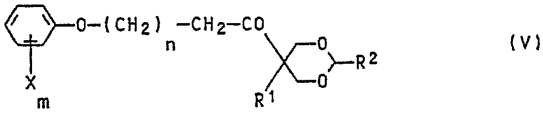 entweder mit Brom in Formamid (H. Bredereck et al., Chem. Ber. 93, 2083 (1960)), mit Dioxan-Dibromid (S.J. Pasariber und L.R. Williams, Aust. J. Chem. 26, 1327 (1973)) oder mit dem Komplex (Pyrrolidon)₃ .HBR . Br₂ (D.C. Awang et al., Can. J. Chem. 47, 706 (1969)) erhalten werden.

Die 1-(1,3-(1,3-Dioxan-5-yl)-2-(1,2,4-triazolyl-(1))-ethan-1-one der Formel IV können beispielsweise durch Umsetzung von entsprechenden 2-Halogen-1(2,5-dialkyl-1,3-dioxan-5-yl)-ethan-1-onen der Formel 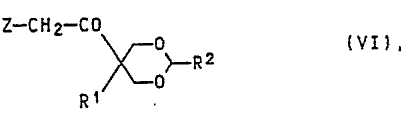 mit 1,2,4-Triazol oder dessen Alkalisalz in einem geeigneten Lösungsmittel erhalten werden.

Die Halogenketone der Formel VI erhält man z.B. durch Bromierung von bekannten (1,3-Dioxan-5-yl)-ethan-1-onen der Formel 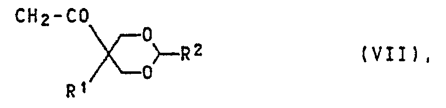 in welcher Rⁱ und R² die oben angegebenen Bedeutungen haben, beispielsweise mit dem Pyrrolidon-Brom-Komplex nach D.P. Wyman und P.R. Kaufmann, J. Org. Chem. 29, 1956 (1964).

Die gemäß a) oder b) erhaltenen Ketone lassen sich stereoselektiv reduzieren, und zwar in bekannter Weise
a) mit sekundären Alkoholaten (EP-A 11 191) oder
b) mit Alkylmagnesiumhalogeniden, die einen Alkylrest mit 2 bis 6 C-Atomen und einem betaständigen Wasserstoffatom im Alkylrest enthalten (vgl. O.Z. 0050/36557) oder
c) mit Wasserstoff in Gegenwart von Ruthenium oder Rutheniumderivaten, insbesondere Rutheniumoxidhydrat (vgl. O.Z. 0050/36557).

Die resultierenden Alkohole der Formel enthalten einen erheblich höheren Anteil der Diastereomeren mit R^{*},S^{*-}Konfiguration als derjenigen mit R^{*},R^{*-}Konfiguration. Der Anteil der R^{*},R^{*-}Diastereomeren im Gemisch liegt in der Regel weit unter 30%. Reine R^{*},S^{*-}Diastereomeren können daraus durch einfaches Waschen der Rohprodukte mit geeigneten Lösungsmitteln, z.B. Diisopropylether, durch Umkristallisieren oder durch andere, übliche Reinigungsschritte, z.B. Chromatographie, erhalten werden.

Bei der - an sich ebenfalls bekannten - Methode der diastereoselektiven Reduktion der Ketone mit komplexen Hydriden, bevorzugt Natriumborhydrid, enthalten die resultierenden Alkohole einen erheblich höheren Anteil der Diastereomeren mit R^{*},R^{*-}Konfiguration.

### Beispiel 1

### a) Herstellung eines Vorprodukts

aa) Zu einer Lösung von 144 g (1 mol) 5-Acetyl-5-methyl-1,3-dioxan und 85,5 g (1 mol) Pyrrolidon in 500 ml Tetrahydrofuran wird bei 50_{°}C innerhalb von 2 Stunden eine Lösung von 498 g (1 mol) Pyrrolidon-Brom-Komplex in 1 I Tetrahydrofuran zugetropft. Nach achtstündigem Nachrühren bei 50_{°}C wird der weiße Niederschlag von Pyrrolidon-Hydrobromid abgesaugt, mit 50 ml Tetrahydrofuran gewaschen und das Filtrat im Vakuum eingeengt. Man erhält 220 g (99%) an 1-(5-Methyl-1,3-dioxan-5-yl)-2-brom-ethan-1-on als öliges Rohprodukt.

ab) Zu einer unter Stickstoff gehaltenen Suspension von 110,1 g (1,1 mol) Natrium-1,2,4-triazolid in 300 ml trockenem Tetrahydrofuran wird innerhalb von 2 Stunden bei 25_{°}C eine Lösung von 223 g (1 mol) 1-(5-Methyl-1,3-dioxan-5-yl)-2-brom-ethan-1-on in 200 ml Tetrahydrofuran zugetropft. Nach achtstündigem Erhitzen unter Rückfluß wird der anorganische Niederschlag abfiltriert und das Filtrat zur Hälfte eingeengt.

Das Gemisch wird angeimpft und über Nacht bei +3_{°}C stehengelassen. Der Niederschlag wird abgesaugt, zuerst mit 30 ml kaltem (+5°C) Tetrahydrofuran, dann mit 80 ml Ether und anschließend mit 100 ml n-Pentan gewaschen und getrocknet. Man erhält 184 g (87,2%) 1-(5-Methyl-1,3-dioxan-5-yl)-2-(1,2,4-triazotyt-(1))-ethan-1-on in Form weißer Kristalle mit einem Schmelzpunkt von 95 bis 97°C.

### b) Herstellung eines Endprodukts (Verbindung Nr. 1 der Tabelle)

Zu einer unter Stickstoff gehaltenen Suspension von 5,3 g (0,22 mol) Natriumhydrid in 50 ml absolutem Dimethylformamid wird unter Rühren bei 20-25°C eine Lösung von 42,2 g (0,2 mol) 1-(5-Methyl-1,3-dioxan-5-yl)-2-(1,2,4-triazolyl-(1))-ethan-1-on in 150 ml absolutem Dimethylformamid zugetropft. Nach dreistündigem Nachrühren wird bei 60°C die Lösung von 49,4 g (0,2 mol) 1-Brom-4-(2-fluorphenoxy)-butan in 50 ml absolutem Dimethylformamid zugetropft. Anschließend wird das Reaktionsgemisch weitere 5 Stunden unter Rückfluß erhitzt. Bei Raumtemperatur werden dann etwa 100 ml Eiswasser vorsichtig zugetropft. Es wird dreimal mit je 300 ml Methylenchlorid extrahiert. Die organische Phase wird anschließend mehrmals mit Wasser gewaschen, über Na₂S0₄ getrocknet und eingeengt. Nach der Destillation (Kpo,oo5 = 248°C) erhält man 56 g (74%) 1-(5-Methyl-1,3-dioxan-5-yl)-2-(1,2,4-triazol-1-yl)-6-(2-fluor- phenoxy)-hexan-1-on als gelbliches Harz.

### Beispiel 2

### (1-R-,2-R-)-1-(5-Methyl-1,3-dioxan-5-yl)-2-(1,2,4-triazol-1-yl)-6-(2-fluorphenoxy)-hexan-l-ol

### (Verbindung Nr. 2 der Tabelle)

Zu einer Lösung von 24,5 g (0,065 mol) 1-(5-Methyl-1,3-dioxan-5-yl)-2-(1,2,4-triazol-1-yl)-6-(2-flu- orophenoxy)-hexan-1-on in 300 ml Methanol werden zwischen 0 und +5°C 3,0 g (0,078 mol) Natriumborhydrid portionsweise zugegeben. Nach fünfstündigem Rühren unter Rückfluß wird eingeengt, der Rückstand zwischen 400 ml Ether und 200 ml Wasser verteilt, die etherische Lösung dreimal mit 200 ml Wasser gewaschen, über Natriumsulfat getrocknet und wiederum eingeengt. Es wird über eine Kieselgelsäule mit Methylenchlorid/Aceton (9:1) chromatographiert und das Lösungsmittel entfernt. Man erhält 13,2 g (54 %) des diastereomerenreinen Produktes als weiße Kristallmasse (Fp. = 87 bis 89°C).

### Beispiel 3

### (1-R-,2-S-)-1-(5-Methyl-1,3-dioxan-5-yl)-2-(1,2,4-triazol-1-yl)-6-(2-fluorphenoxy)-hexan-l-ol

### (Verbindung Nr. 3 der Tabelle)

Unter Stickstoff tropft man zur Lösung von ca. 0,06 mol auf die übliche Weise hergestelltem n-Propylmagnesiumbromid in 100 ml trockenem Tetrahydrofuran bei Rückflußtemperatur unter Rühren eine Lösung von 8,0 g (0,021 mol) 1-(5-Methyl-1 ,3-dioxan-5-yl)-6-(2-fluorphenoxy)-hexan-1-on in 100 ml trockenem Tetrahydrofuran. Man erhitzt die Mischung nach vollständiger Zugabe noch weitere 5 Stunden unter Rückfluß. Anschließend wird bei 0_{°}C tropfenweise mit 50 ml Wasser hydrolysiert und mit 50 ml konzentrierter Ammoniumchloridlösung versetzt. Es wird dreimal mit je 200 ml Diethylether extrahiert, mit Wasser neutral gewaschen und über Magnesiumsulfat getrocknet. Man chromatographiert über eine Kieselgelsäule mit Methylenchlorid/Aceton (9:1) und erhält 3,5 g (44 %) des diastereomerenreinen Produktes als hellgelbes Öl.

¹H-NMR (80 MHz/CDCI): 8 = 0,9 (s, 3H), 1,0-1,4 (m, 2H), 2,8 (m, 2H), 2,0-2,1 (m, 2H), 3,4 (2d, 2H), 3,8-4,2 (m, 6H), 4,6-4,7 (m, 2H), 4,95 (d, 1 H), 6,8-7,1 (m, 4H), 7,95 (s, 1 H), 8,15 (s, 1 H).

Als Erkennungsmerkmal für die R^{*},R^{*-}Diastereomeren kann z.B. dienen, daß im ¹⁻H-NMR-Spektrum das Signal für die 3 Protonen der Methylgruppe am Dioxanylrest bei 0,1 bis 0,2 ppm auftritt, während es beim R^{*},S^{*-}Diastereomeren bei 0,8 bis 1,0 ppm liegt.

Unter entsprechender Abwandlung der vorstehenden Angaben wurden die in der folgenden Tabelle aufgeführten Verbindungen hergestellt (Dia A = R^{*},R^{*-}Diastereomeres; Dia B = R^{*},S^{*-}Diastereomeres).

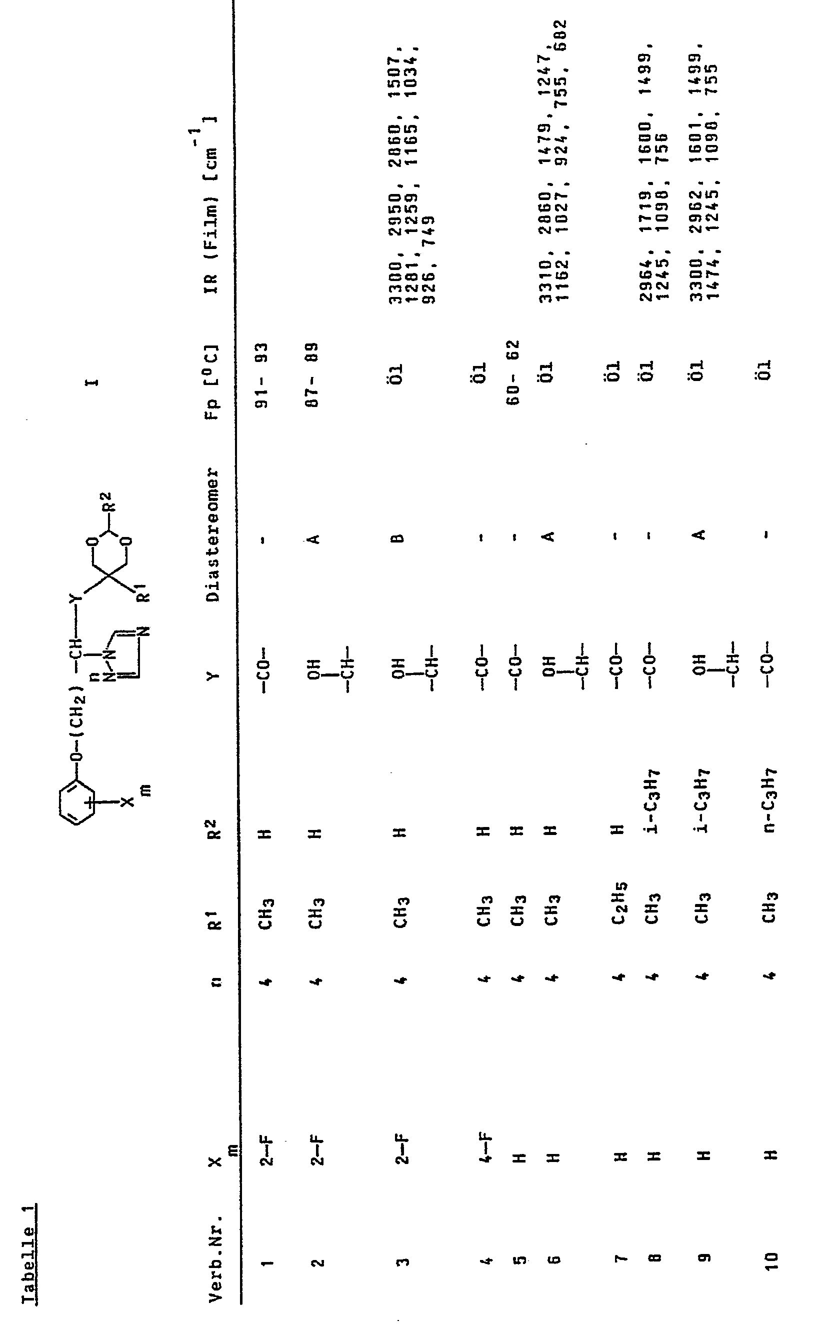

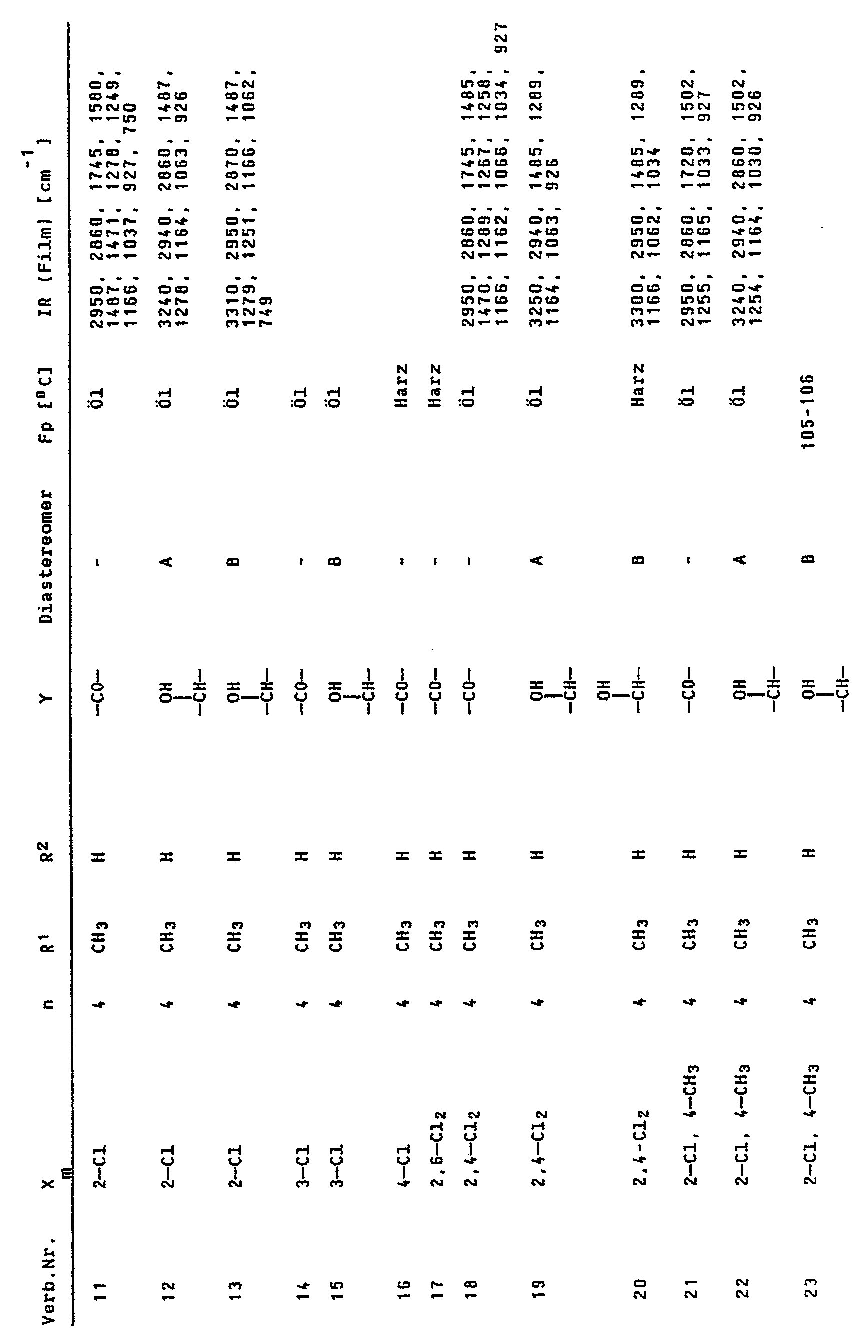

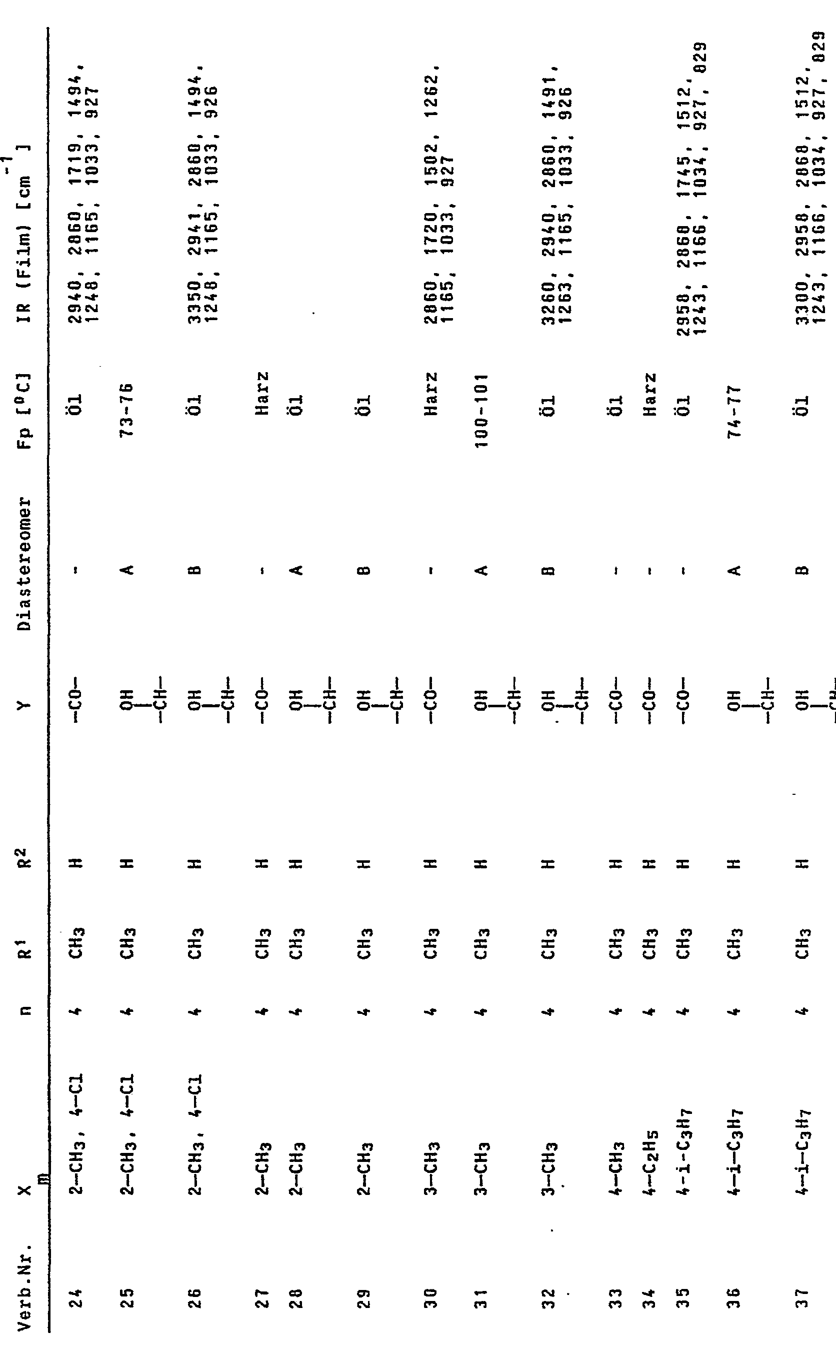

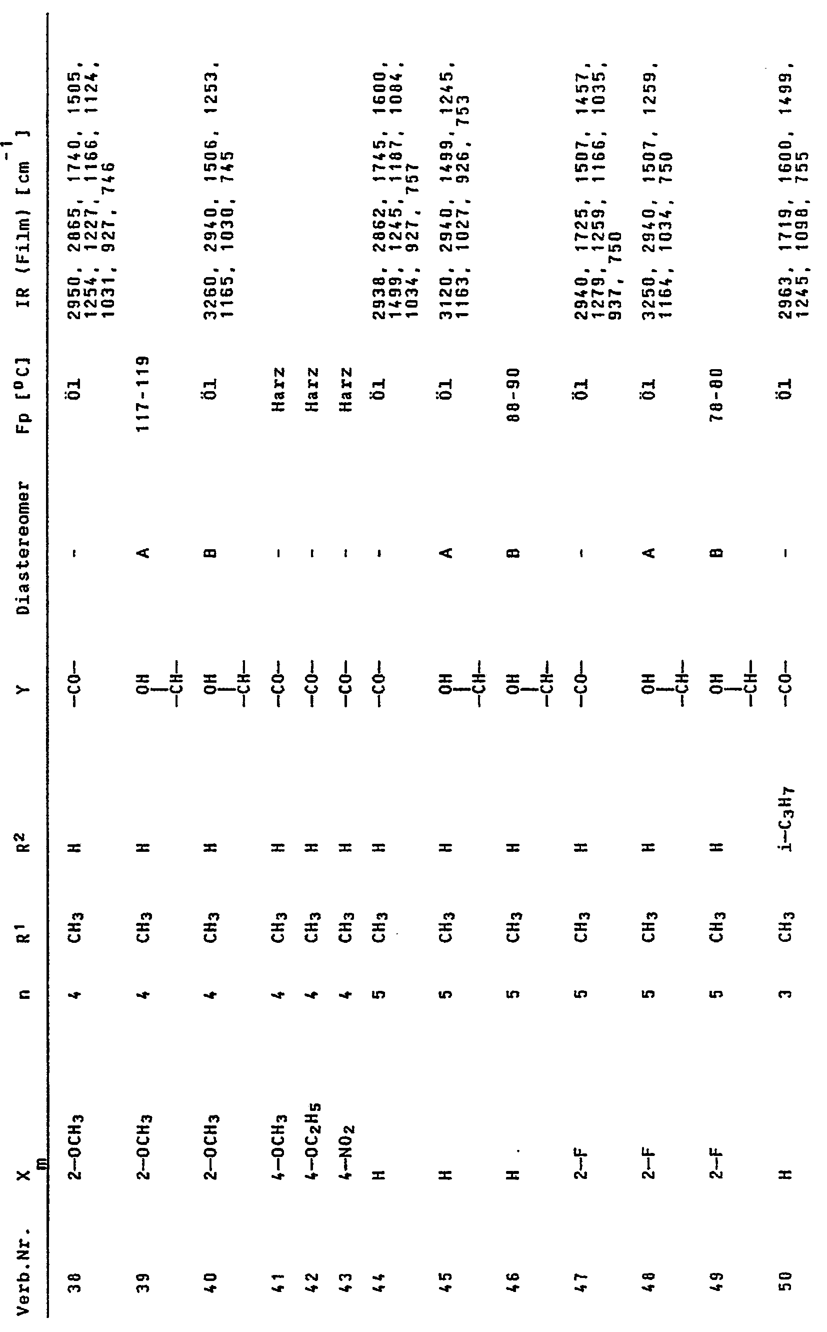

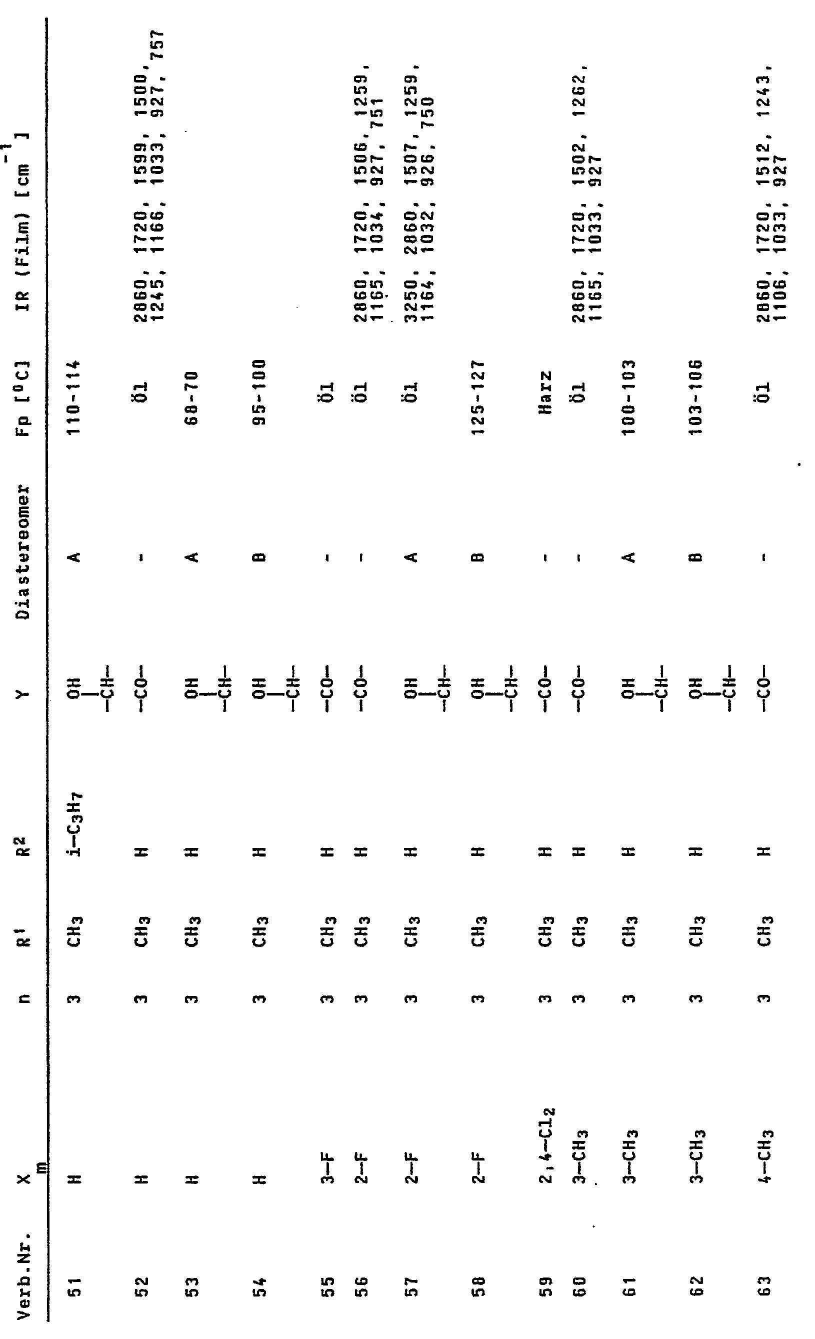

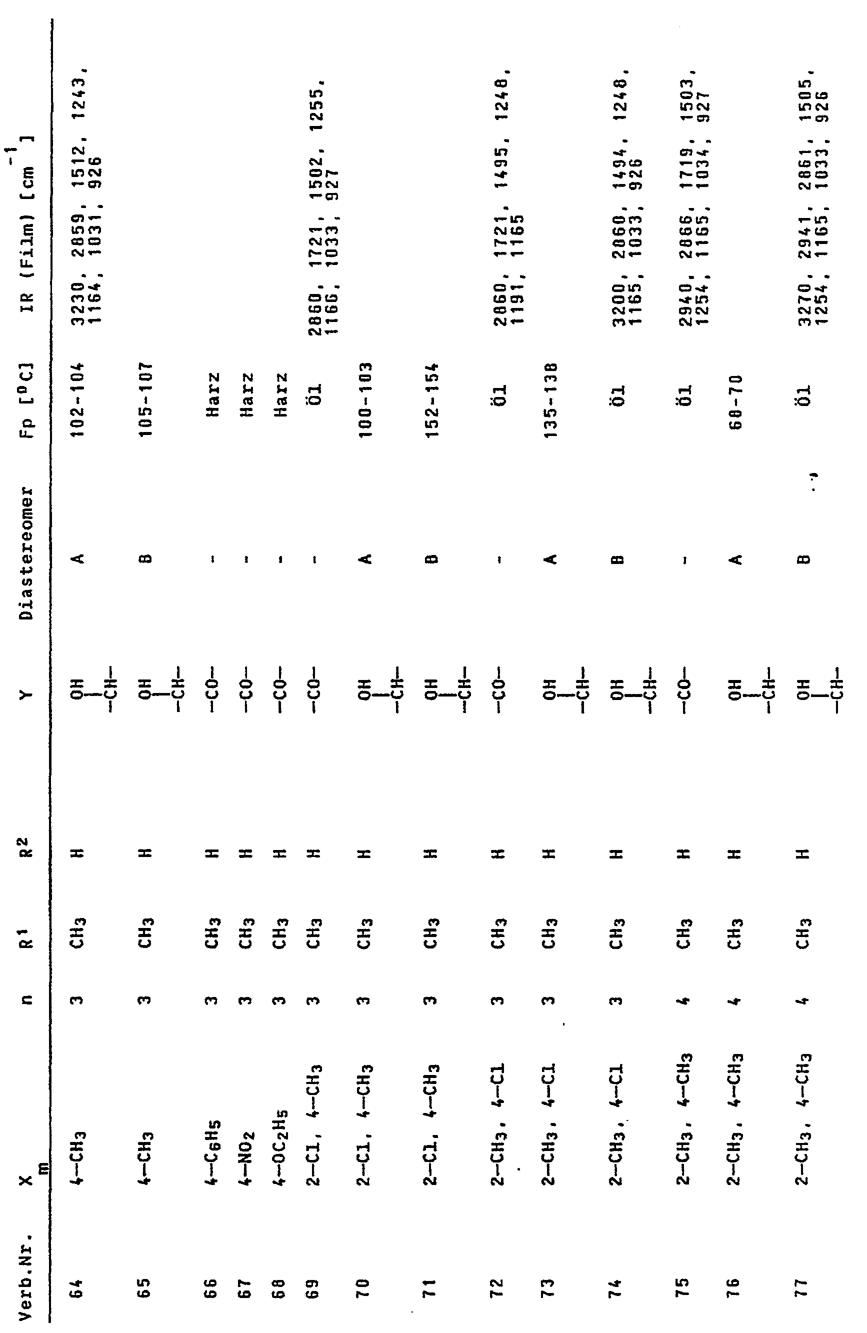

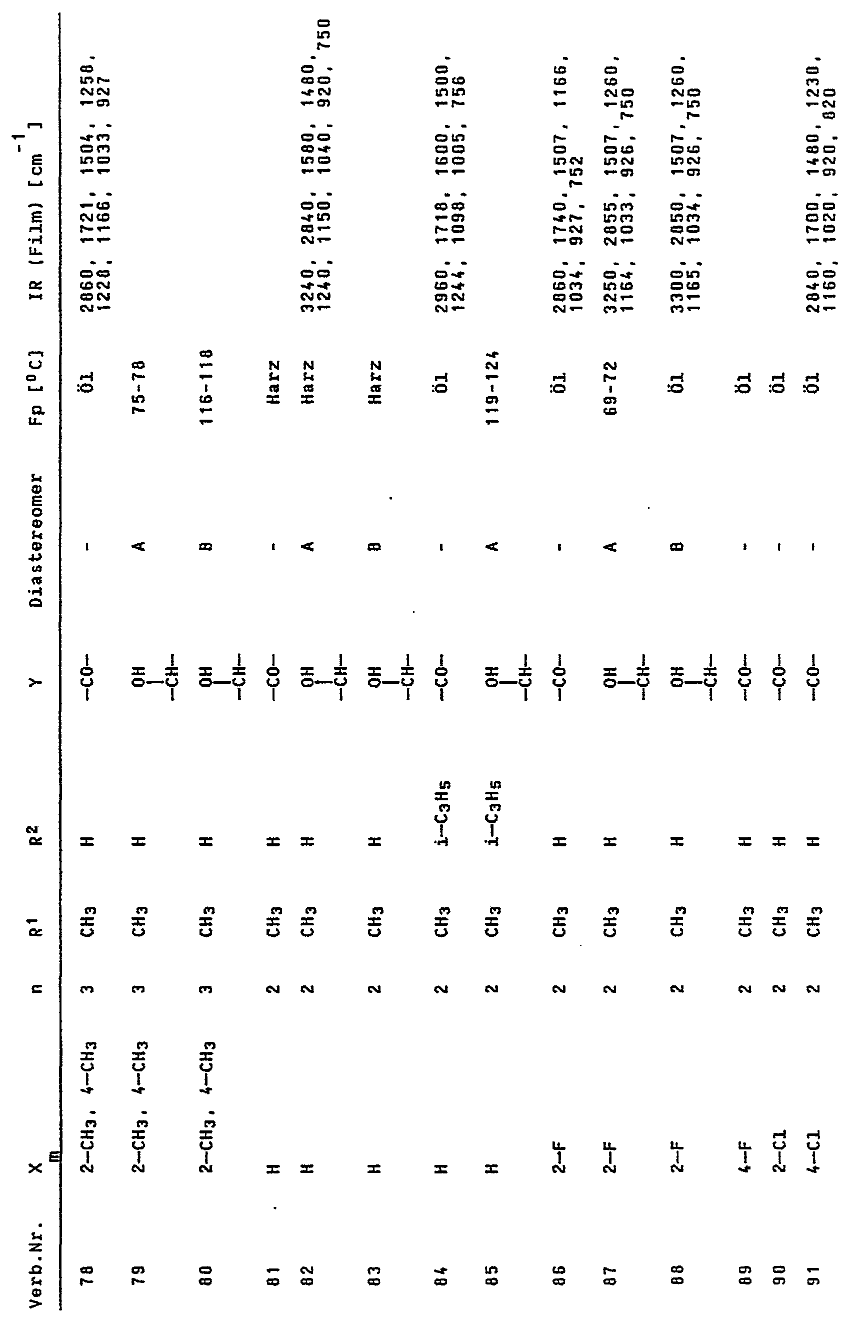

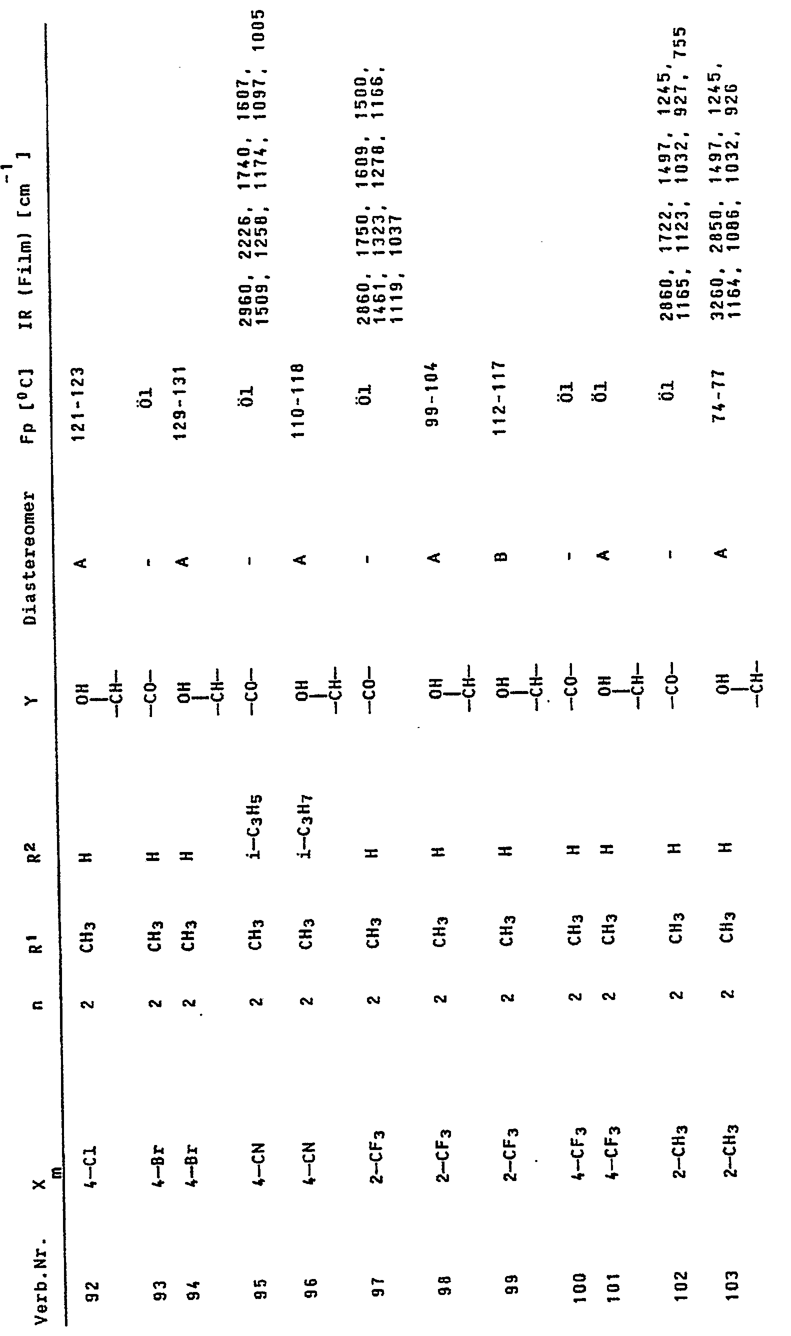

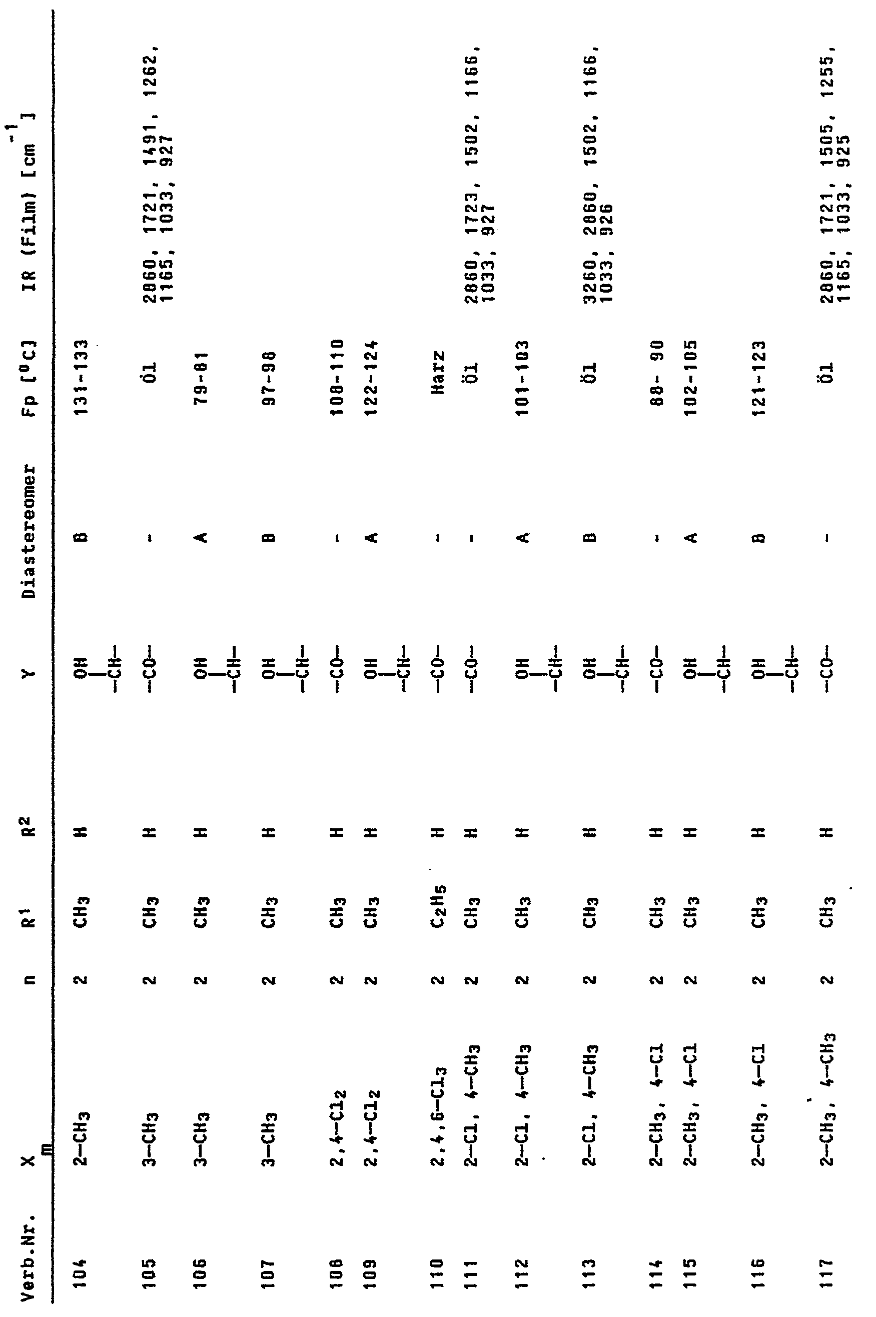

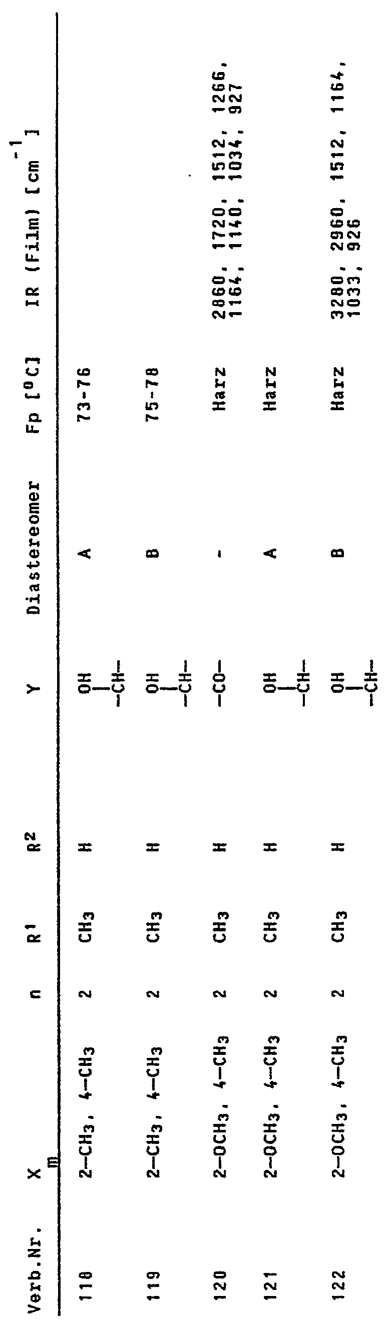

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanzen dienten Chlorcholinchlorid und die nachstehend mit B, C und D bezeichneten Wirkstoffe.

Gleichlaufend zur Reduzierung des Längenwachstums stieg die Farbintensität der Blätter an. Der erhöhte Chlorophyllgehalt läßt eine ebenfalls erhöhte Photosyntheserate und damit erhöhte Ertragsbildung erwarten.

Vergleichssubstanzen: 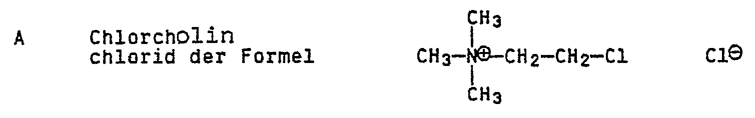 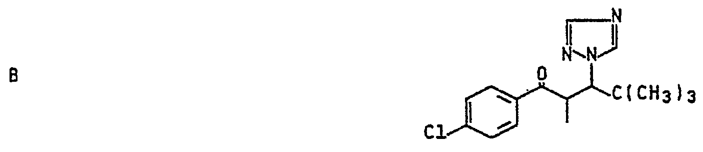 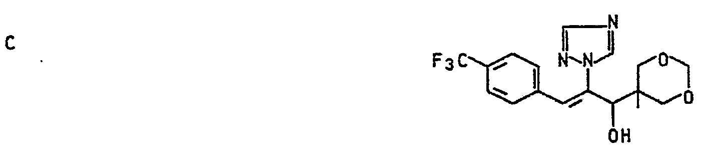 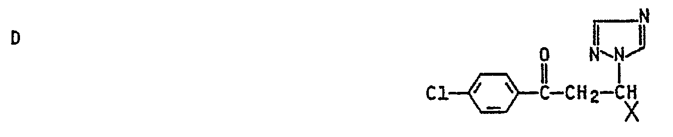 Dabei ergab sich, daß in Versuchen mit Sommergerste, Sonnenblumen und Sommerraps sowie im Reiskeimlingstest hinsichtlich Standfestigkeit und Wuchshöhenreduzierung bei Aufwandmengen von 1,5 und 6 mg Wirkstoff je Gefäß die Verbindungen 3, 5, 8, 9, 50, 51, 82, 84, 85, 92, 95, 96, 108, 109, 114 und 115 durchwegs eine günstigere Wirkung als der Vergleich erzielten.

## Patentansprüche

1. Verbindung der Formel 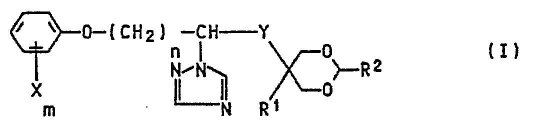 wobei
R1 und R² verschieden oder gleich sind und Wasserstoff oder Alkyl mit 1 bis 5 C-Atomen bedeuten,
X ( m = null bis 5) gleiche oder verschiedene Substituenten, ausgewählt aus Halogen, Cyano, Trifluormethyl, Nitro, G-C4-Alkyl, Cₗ-C₄-Alkoxy, Phenyl oder Wasserstoff bedeuten,
n eine ganze Zahl von 2 bis 6 und
Y die -CO- oder die -CH(OH)-Gruppe bedeutet.

2. Verbindungen der Formel I gemäß Anspruch 1, wobei X: Wasserstoff, 2-Fluor-, 4-Fluor-, 2-Chlor-, 3-Chlor-, 4-Chlor-, 4-Brom-, 2,4-Dichlor-, 2,6-Dichlor-, 3,4-Dichlor-, 2,4,6-Trichlor-, 2-Chlor-4-methyl-, 2-Methyl-4-chlor-, 2-Methyl-, 3-Methyl-, 4-Methyl-, 4-Ethyl-, 4-lsopropyl-, 4-tert.-Butyl-, 2,4-Dimethyl-, 2,4,6-Trimethyl-, 2-Methoxy-4-methyl-, 2-Methoxy-, 4-Methoxy-, 4-Ethoxy-, 2-Trifluormethyl-, 4-Trifluormethyl-, 4-Cyano-, 4-Nitro-, 4-Phenyl-; R¹: Methyl oder Ethyl und R²: Wasserstoff, Methyl, Ethyl, n-Propyl, Isopropyl, n-Butyl, sek.-Butyl, Isobutyl, tert.-Butyl, n-Pentyl oder Neopentyl bedeutet.

3. Verfahren zur Herstellung von Verbindungen der Formel I, dadurch gekennzeichnet, daß man in an sich bekannter Weise entweder
a) ein 1,2,4-Triazol mit einem entsprechenden a-Bromketon der Formel 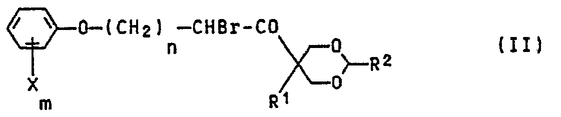 oder
b) ein entsprechendes Aryloxyalkylhalogenid der Formel 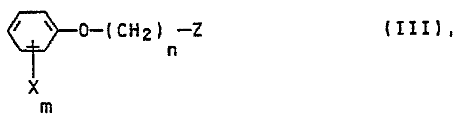 wobei Z Halogen (Chlor oder Brom) ist, mit einem entsprechenden 1-(1,3-Dioxan-5-yl)-2-(1,2,4-triazolyl)-ethan-1-on der Formel 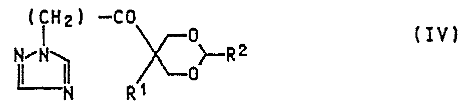 umsetzt.

4. Verbindungen der Formel 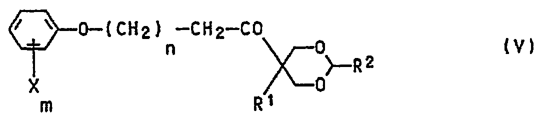 in der Ri, R2 und X die in Anspruch 1 angegebene Bedeutung haben sowie deren Bromierungsprodukt der Formel gemäß Anspruch 3.

5. Mittel zur Regulierung des Pflanzenwachstums, enthaltend eine oder mehrere Verbindungen der Formel I gemäß Anspruch 1.

6. Mittel zur Regulierung des Pflanzenwachstums, enthaltend eine oder mehrere Verbindungen der Formel I gemäß Anspruch 1 und einen flüssigen oder festen Trägerstoff sowie gegebenenfalls eine oder mehrere oberflächenaktive Mittel.

7. Verwendung einer Verbindung der Formel gemäß Anspruch 1 zur Regulierung des Pflanzenwachstums.

8. Verfahren zur Regulierung des Pflanzenwachstums, dadurch aekennzeichnet, daß man eine oder mehrere Verbindungen der Formel 1 gemäß Anspruch 1 auf Pflanzen oder deren Lebensraum einwirken läßt.

9. Verfahren zur Herstellung von das Pflanzenwachstum regulierenden Mitteln, dadurch gekennzeichnet, daß man eine oder mehrere Verbindungen der Formel gemäß Anspruch 1 mit festen oder flüssigen Trägerstoffen sowie gegebenenfalls einem oder mehreren oberflächenaktiven Mitteln mischt.

## Claims

1. A compound of the formula I
where R¹ and R² are identical or different and are each hydrogen or alkyl of 1 to 5 carbon atoms, the radicals X are identical or different substituents selected from the group consisting of halogen, cyano, trifluoromethyl, nitro, Cₗ-C₄-alkyl, Cₗ-C₄-alkoxy, phenyl or hydrogen, m is from zero to 5, n is an integer from 2 to 6 and Y is -CO- or -CH(OH)-.

2. A compound of the formula I as claimed in claim 1, wherein X is hydrogen, 2-fluoro-, 4-fluoro-, 2-chloro-, 3-chloro-, 4-chloro-, 4-bromo-, 2,4-dichloro-, 2,6-dichloro-, 3,4-dichloro-, 2,4,6-trichloro-, 2-chloro-4-methyl-, 2-methyl-4-chloro-, 2-methyl-, 3-methyl-, 4-methyl-, 4-ethyl-, 4-isopropyl-, 4-tert-butyl-, 2,4-dimethyl-, 2,4,6-trimethyl-, 2-methoxy-4-methyl-, 2-methoxy-, 4-methoxy-, 4-ethoxy-, 2-trifluoromethyl-, 4-trifluoromethyl-, 4-cyano-, 4-nitro- or 4-phenyl-, R¹ is methyl or ethyl and R2 is hydrogen, methyl, ethyl, n-propyl, isopropyl, n-butyl, sec-butyl, isobutyl, tert-butyl, n-pentyl or neopentyl.

3. A process for the preparation of a compound of the formula I, wherein either
a) a 1,2,4-triazole is reacted with an appropriate a-bromoketone of the formula II or
b) an appropriate aryloxyalkyl halide of the formula III wherein Z is halogen (chlorine or bromine), is reacted with an appropriate 1-(1,3-dioxan-5-yl)-2-(1,2,4-triazolyl)-ethan-1-one of the formula IV the reactions being carried out in a conventional manner.

4. A compound of the formula V
where R¹, R² and X have the meanings stated in claim 1, and its bromination product of the formula II as shown in claim 3.

5. A plant growth regulator containing one or more compounds of the formula I as claimed in claim 1.

6. A plant growth regulator containing one or more compounds of the formula I as claimed in claim 1 and a liquid or solid carrier and, if required, one or more surfactants.

7. Use of a compound of the formula I as claimed in claim 1 for regulating plant growth.

8. A method of regulating plant growth, wherein one or more compounds of the formula I as claimed in claim 1 are allowed to act on plants or their habitat.

9. A process for the preparation of a plant growth regulator, wherein one or more compounds of the formula I as claimed in claim 1 are mixed with solid or liquid carriers and, if required, with one or more surfactants.

## Revendications

1. Composé de formule 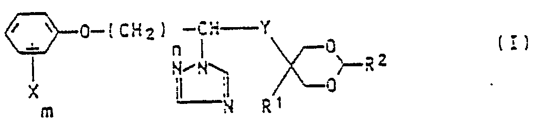 où
Ri et R₂ sont identiques ou différents et représentent hydrogène ou alkyle à 1 à 5 atomes C
X (m=0 à 5) représente des substituants identiques ou différents, choisis parmi halogène, cyano, trifluorométhyl, nitro, alkyle en Ci-C₄ alcoxy en Ci-C₄, phényle ou hydrogène
n représente un nombre entier de 2 à 6 et
Y le groupe -CO- ou le groupe -CH(OH)-.

2. Composés de formule 1 selon la revendication 1, où X représente: hydrogène, 2-fluoro-, 4-fluoro-, 2-chloro-, 3-chloro-, 4-chloro-, 4-bromo-, 2,4-dichloro-, 2,6-dichloro-, 3,4-dichloro-, 2,4,6-trichloro-, 2-chloro-4-méthyl-, 2-méthyl-4-chloro-, 2-méthyl-, 3-méthyl-, 4-méthyl-, 4-éthyl-4-isopropyl-, 4-tert.-butyl-, 2,4-diméthyl-, 2,4,6-triméthyl-, 2-méthoxy-4-méthyl-, 2-méthoxy-, 4-méthoxy-, 4-éthoxy-, 2-trifluorométhyl-, 4-trifluorométhyl-, 4-cyano-, 4-nitro-, 4-phényl-; R¹: méthyle ou éthyle et R²: hydrogène, méthyle, éthyle, n-propyle, isopropyle, n-butyle, sec.-butyle, isobutyle, tert.-butyle, n-pentyle ou néopentyle.

3. Procédé de préparation de composés de formule I, caractérisé par le fait que l'on fait réagir, de manière connue en soi, soit:
a) un 1,2,4-triazole avec une a-bromocétone de formule 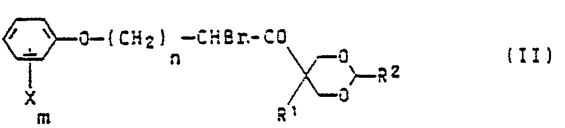
b) un halogénure d'aryloxyalkyle correspondant de formule III 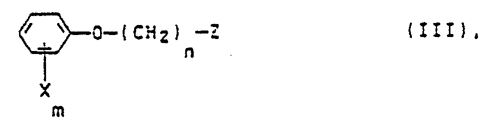 où Z est halogène (chlore ou brome) avec une 1-(1,3-dioxan-5-yl)-2-(1,2,4-triazolyl)-éthan-1-one correspondante de formule

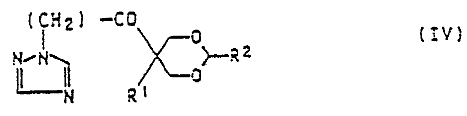

4. Composés de formule 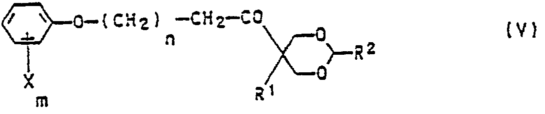 dans laquelle R¹, R² et X ont la signification donnée dans la revendication 1, ainsi que leur produit de bro- mation de formule Il selon la revendication 3.

5. Moyen de régulation de la croissance des plantes, contenant un ou plusieurs composés de formule I selon la revendication 1.

6. Moyen de régulation de la croissance des plantes, contenant un ou plusieurs composés de formule 1 selon la revendication 1 et un support liquide ou solide ainsi qu'éventuellement un ou plusieurs agents tensioactifs.

7. Utilisation d'un composé de formule I selon la revendication 1 pour la régulation de la croissance des plantes.

8. Procédé de régulation de la croissance des plantes, caractérisé par le fait qu'on fait agir un ou plusieurs composés de formule I selon la revendication 1 sur des plantes ou leur biotope.

9. Procédé de préparation de moyens de régulation de la croissance des plantes, caractérisé par le fait qu'on mélange un ou plusieurs composés de formule 1 selon la revendication 1 avec des supports liquides ou solides ainsi qu'éventuellement un ou plusieurs agents tensioactifs.
